# EUROPEAN PATENT APPLICATION

(11) **EP 2 201 917 A1**
(43) Date of publication of application: **30.06.2010**
(21) Application number: 09252880.1
(22) Date of filing: 22.12.2009
(51) Int. Cl.: A61F 7/02

(54) **Thermal treatment device**

(30) Priority: 23.12.2008 US 140146 P
(71) Applicant: McNeil-PPC, Inc., Skillman, NJ 08558 (US)
(72) Inventor: Lynch, Joseph M., Fort Washington, PA 19034 (US); Sowden, Harry S., Glenside, PA 19038 (US); Robinson, Ronni L., Ambler, PA 19002 (US); Wiet, Stephan G., Morristown, NJ 07960 (US); Kriksunov, Leo B., Glenside, Pennsylvania 19038 (US)
(74) Representative: Kirsch, Susan Edith

(57) **Abstract**

A thermal treatment device to be worn in close proximity to the skin of a human is disclosed. The thermal treatment device comprises a thermal composition; wherein the device substantially covers the left or right trapezius muscle of the neck and shoulder or the left or right paraspinal muscles in the lower back and the tender point of the respective sacroiliac joint.

## Description

### BACKGROUND OF THE INVENTION

### Field of the Invention

The present invention is directed to a thermal treatment device. More particularly, the present invention relates to a thermal treatment device that is contoured to cover the surface of the trapezius muscle or paraspinal muscles of the lower back.

### Related Background Art

For patients with aching muscles and sore joints, the application of heat can decrease the viscosity of fluids, loosen stiff muscles, improve blood flow to the affected area, facilitate tissue repair, and create a feeling of relaxation. The application of heat to the skin as a means to penetrate deeper into tissues has historically been used for pain relief of muscles and joints, as well as for the treatment of certain inflammatory conditions. For some acute injuries, the application of cold can numb pain, constrict blood vessels and mitigate the inflammatory response. The application of cold materials to the skin has also been used for similar treatments, especially for treating inflammatory responses such as joint inflammation.

Traditional heating devices have, in some instances, generated heat using chemical formulations, such as iron powder formulations, that oxidize when exposed to air. Commercially available thermal chemical formulation products are mainly categorized with disposable heat patches, which are available as loosely formed fabric thermally active components filled with the exothermic composition. An alternate means of providing heat is by way of electrical heating elements that are attached to a power source. Since the desired time of use is often longer than 4 hours, in the case of an electrical source, the power source typically used in these types of devices is either an electrical wall outlet or a battery.

Other chemical heating devices include those products that incorporate heating portions into fabrics that can conform or are shaped to fit various parts of the body, such as the knee or the back as shown in U.S. Patent No. 6,074,413. In these cases, typically the entire product, including the garment and the heat providing exothermic formulation materials, are disposable because they are incorporated into a unitary product. The chemical heating portion is not removable from such a unitary product, and therefore, the entire device is designed to be disposed following use. Each use can typically last for 6 to 12 hours, and a user can use 2-3 of these products over a 24-hour period. These types of products have the disadvantage of having loose powder formulations that do not always adequately conform to parts of the skin and do not conduct heat thoroughly to the skin since a woven or non-woven fabric surface is in contact with the skin.

Other types of devices, such as those shown in U.S. Patent No. 5,484,366, exemplify elements that are not disposable, such as using a back belt with gel insert containers. In such a device the gel-inserts must be manually re-heated or cooled, taking more active participation by the user in order to be reusable. Similarly, the device shown in U.S. Patent No. 6,416,534 uses a back belt with a flexible fabric, and a gel insert that is reheated using electrical heat. This type of device also involves active participation on the part of the user and a potential lag time in order to heat the gel-insert. U.S. Patent No. 6,074,413 is directed to a disposable thermal back wrap having one or more thermal inserts comprising a plurality of heat cells, wherein heat is applied to specific areas of the user's back, for pain relief. U.S. Patent No. 5,605,144 is directed to a heating garment with pouch for accommodating inserted chemical heating inserts that are air activated.

U.S. Patent No. 5,484,366 is directed to an aerobic/cross training exercise belt. The belt comprises a straight piece of material having a fastener on each end whereby the ends can be fastened together to form a closed belt. A back lumbar support is connected to the rear body of the belt. The back lumbar support has at least one pocket to mount chemical gel-inserts whereby the user would have a thermal application to the lumbar area while wearing the belt. The gel inserts can be heated or cooled to the desired temperature. U.S. Patent No. 6,623,419 is directed to a therapeutic back belt and related method of manufacture. The belt includes magnets that are secured to the belt and thermally active gel material. U.S. Patent No. 5,179,942 is directed to a lumbar support therapeutic heat/cooling/air belt. The support has one pocket in the lower back section that is capable of receiving an insert to create a thermal change or provide air for support purposes.

Additional devices have also been disclosed, as shown in U.S. Patent No. 7,147,610, that incorporate massaging elements with the heating elements so that they are conveniently available in a single device. Such a device involves excess bulk, is non-discreet and requires the use of external power sources (i.e., a junction box) since the heating and massaging element require the use of electrical power. In addition, although the parts are reusable, electrical elements tend to be non-washable. Published U.S. Patent Application No. 2004/0082886 is directed to a therapeutic device for relieving pain and stress in the hands and feet. The portable device provides heat and vibratory therapies for the hand or foot.

U.S. Patent No. 5,925,072 is directed to a disposable elastic thermal insert wherein iron powder based exothermic compositions are segmented into individual portions and integrated into a back belt. In this composition, the thermal conductivity is not optimized since the composition is separated from the skin by a fabric barrier. U.S. Patent No. 5,918,590 is directed to a specific heat cell unit comprising an iron powder based exothermic composition, wherein a specific exothermic formulation and pocket fill volume are defined.

U.S. Patent No. 6,146,342 is directed to a massage pad having a plurality of randomly actuated pressure inducing elements. The apparatus massages the body by subjecting the body to impacts from reciprocating plungers. The plungers are secured in a flat array within a flexible pad. Each plunger has an associated solenoid device that alternately causes the plunger to project from the pad and to retract within the pad. An electrical circuit includes a power cord and plug assembly, manual controls disposed serially on the cord and plug assembly, and a controller that generates operating signals randomly to the solenoids. A heating element is optionally included in the flexible pad, with a suitable controller provided among the controls.

Still other types of devices, as shown in U.S. Patent No. 7,077,858, include those that use flexible heat exchangers to distribute cooling and heating agents to the skin utilizing electrical heat. U.S. Patent No. 6,409,748 is directed to a heating pad with removable gel insert that provides rapid initial warming. U.S. Patent No. 4,846,176 is directed to a thermal bandage having a conformable region that can be placed against the skin to uniformly heat or cool the contacted skin area.

There is a need in the art for improved thermal treatment devices for the upper and the lower back.

### SUMMARY OF THE INVENTION

It is an object of the invention to provide a thermal treatment device that can be worn in close proximity to the skin of a human. The thermal treatment device comprises: a thermal composition; wherein the device substantially covers the trapezius muscle or the paraspinal muscles in the lower back and the tender point of the respective sacroiliac joint. The thermal treatment device is convenient in that it permits the manufacturer to manufacture and the consumer to purchase and use a single shape to cover portions of the upper and lower back that are known regions of pain and discomfort as needed.

### BRIEF DESCRIPTION OF THE DRAWINGS

Figures 1A, 1B and 1C depict one embodiment of a thermal treatment device of the present invention substantially shaped as a trapezoid, wherein the thermal treatment device is designed to be worn on the right hand side of the body;

Figure 1D is a cross sectional view of the thermal treatment device depicted in Figures 1A and 1B;

Figures 2A, 2B, and 2C depict another embodiment of a thermal treatment device of the present invention substantially shaped as a trapezoid, wherein the thermal treatment device is designed to be worn on the left hand side of the body;

Figure 2D is a cross sectional view of the thermal treatment device depicted in Figures 2A and 2B;

Figures 3A, 3B, and 3C depict yet another embodiment of a thermal treatment device of the present invention substantially shaped as a trapezoid, wherein the thermal treatment device is designed with raised portions on one surface of the device and is designed to be worn on the right hand side of the body;

Figures 4A, 4B, and 4C depict still yet another embodiment of a thermal treatment device of the present invention substantially shaped as a trapezoid, wherein the thermal treatment device is designed with raised portions on one surface of the device and is designed to be worn on the left hand side of the body;

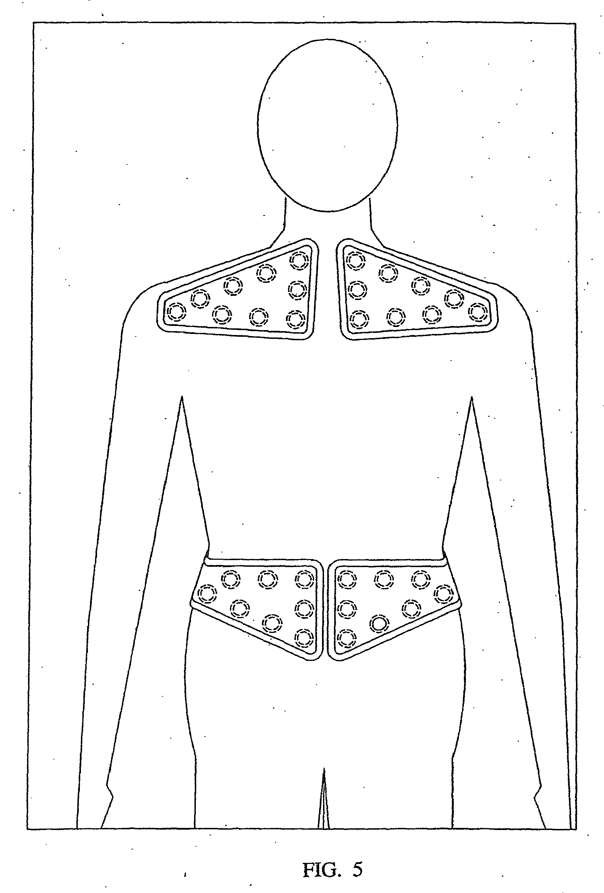

Figure 5 depicts examples of where on the body the thermal treatment device of the present invention substantially shaped as a trapezoid may be worn or placed; and

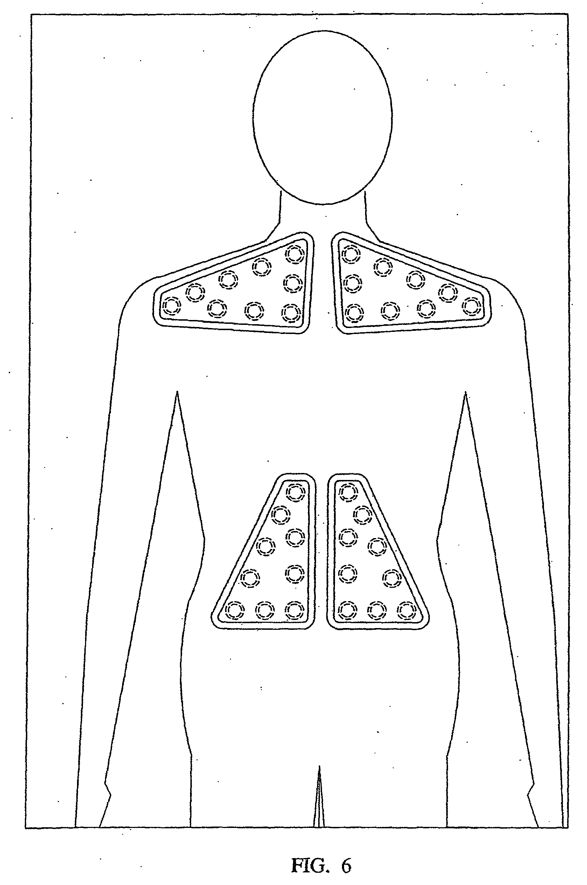

Figure 6 depicts other examples of where on the body the thermal treatment device of the present invention substantially shaped as a trapezoid may be worn or placed.

Figures 7A, 7B, 7C and 7D depict embodiments of a thermal treatment device of the present invention substantially shaped as trapezoidal axisymmetric having various dimensions. Figure 7E depicts the measurement of the dimensions of the device of 7D.

Figure 8A and 8B depict examples of where on the body the thermal treatment device of the present invention substantially shaped as a trapezoidal axisymmetric may be worn or placed.

### DETAILED DESCRIPTION OF THE INVENTION

As used herein, unless otherwise indicated, the term substantially is understood to mean at least about 60%. More preferably, at least about 75%.

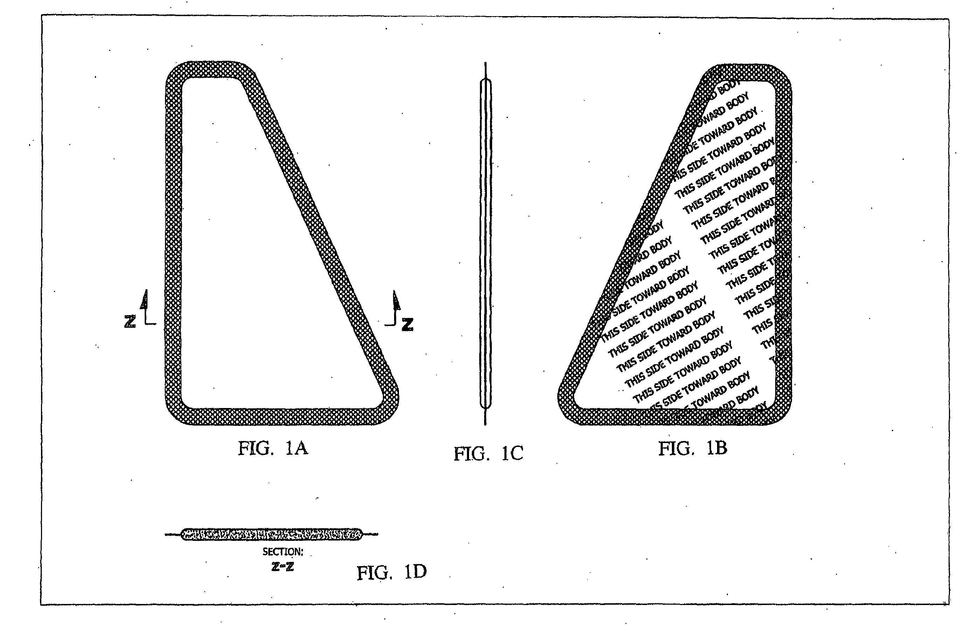

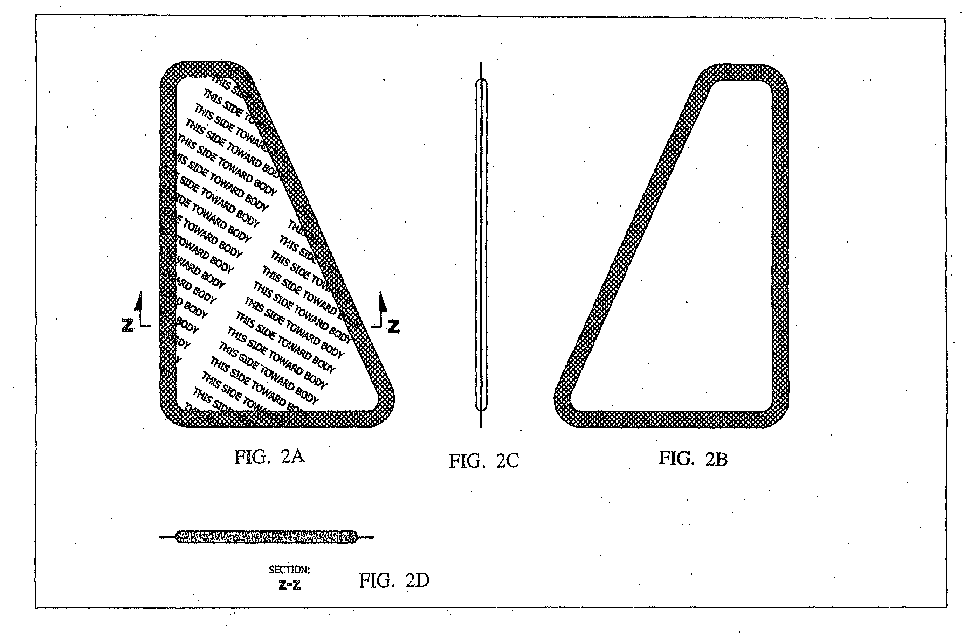

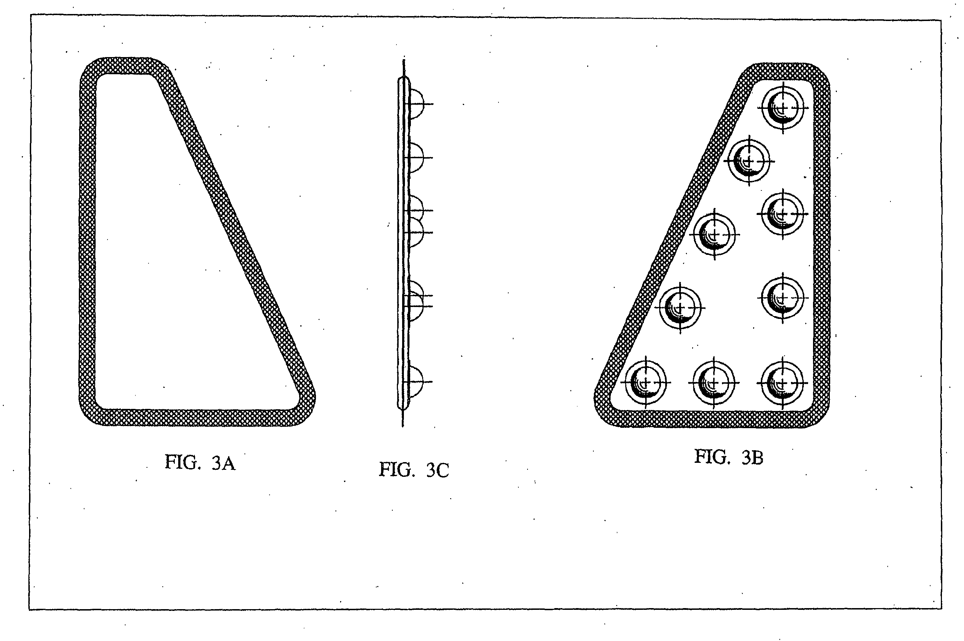

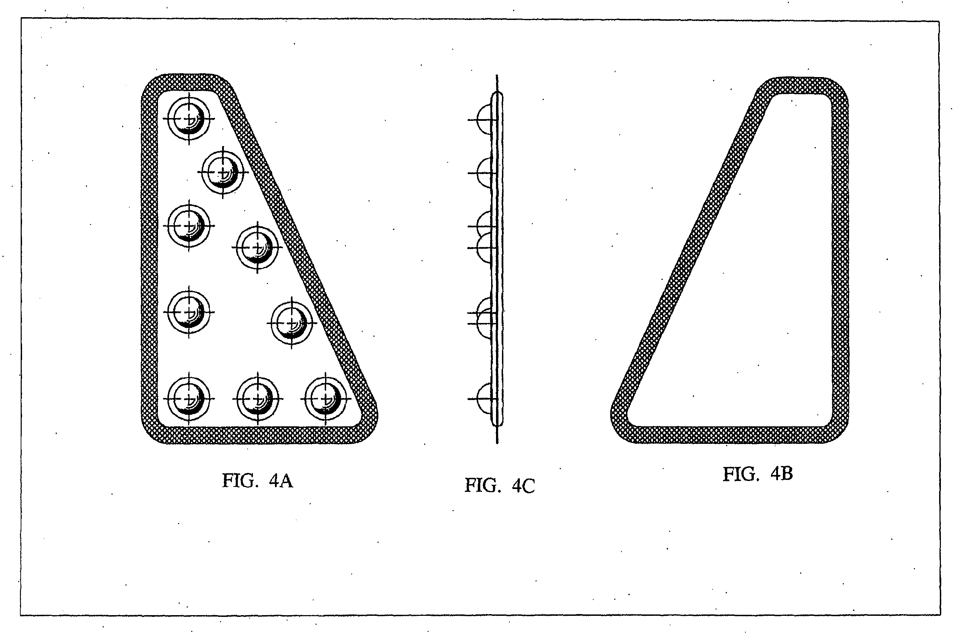

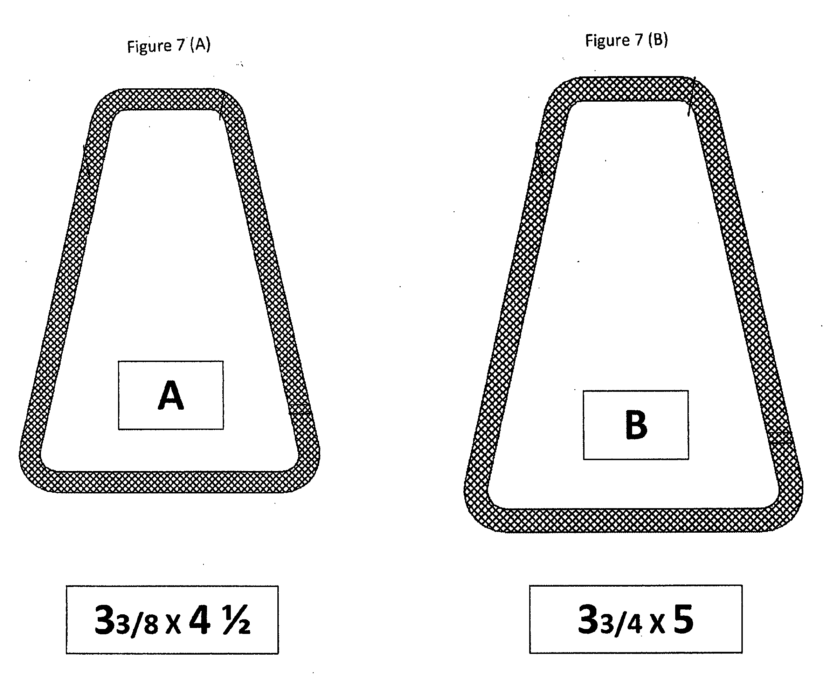

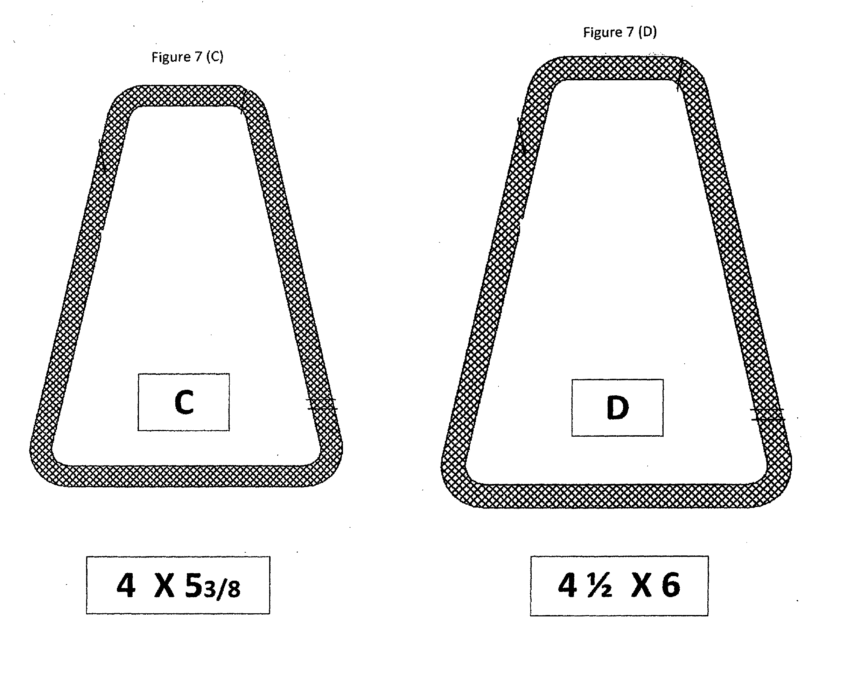

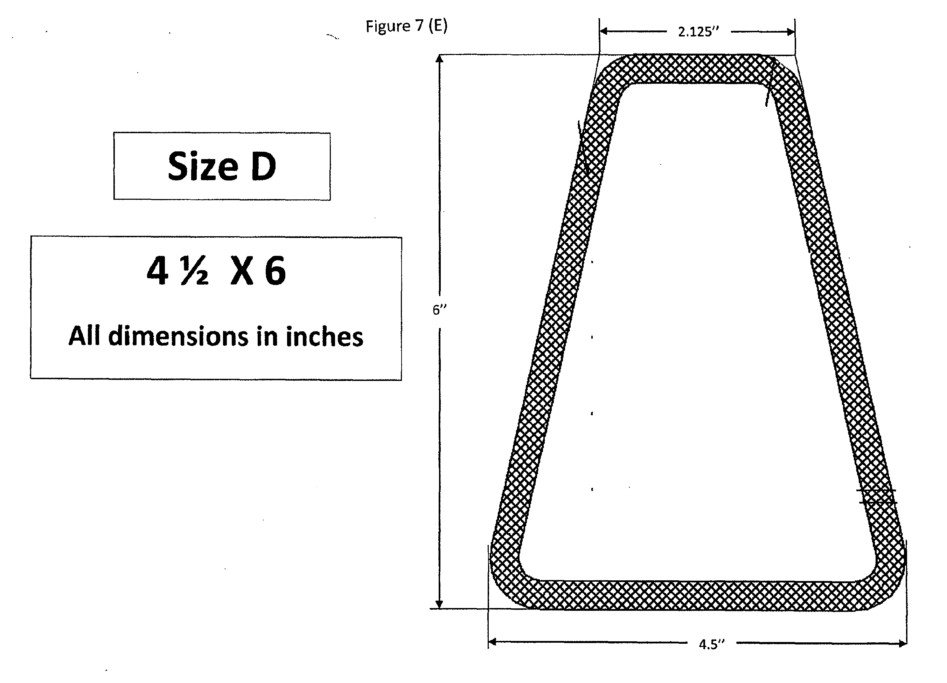

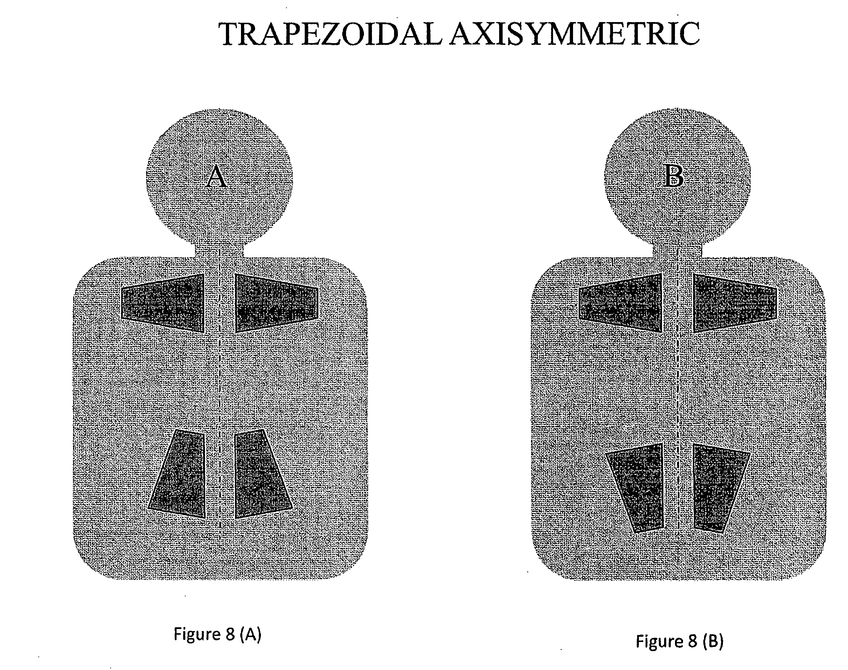

The present invention relates to a thermal treatment device, e.g., thermal pad, for managing muscle and joint pain that is worn in close proximity to the skin of a human. The thermal treatment device offers the benefit of improved therapeutic relief by substantially covering the surface of the affected muscle groups, thereby targeting treatment of those particular muscle groups. By designing the thermal treatment device with the particular shape of a muscle or muscle group, the device can be used for either treating the back, neck or shoulder of the human body. The particular shape and dimensions of the device of the present invention have the added benefit of minimizing waste and manufacturing costs. The thermal treatment device, which may be substantially shaped as a trapezoid, may be designed for use on the right hand side of the body. See Figures 1A, 1B, 3A, and 3B. Alternatively, the thermal treatment device, which may be substantially shaped as a trapezoid, may be designed for use on the left hand side of the body. See Figures 2A, 2B, 4A, and 4B. In one embodiment, the thermal treatment device may be designed for use on either side of the body. For example, the thermal treatment device may by substantially shaped as trapezoidal axisymmetric. See Figures 7A-7E and Figures 8A and 8B.

The device substantially covers the left or right trapezius muscle of the neck and shoulder or the paraspinal muscles in the lower back and the tender point of the respective sacroiliac joint.

The thermal treatment device of the present invention will typically be worn in direct contact with the skin, either on top of a garment or beneath a garment. In one embodiment the thermal treatment device includes an adhesive, which may adhere to a garment or to the skin.

The thermal treatment device of the invention is designed to cover the surface of the left or right trapezius muscle of the neck or shoulder or the paraspinal muscles in the lower back and the tender point of the respective sacroiliac joint. In order to cover the surface of the desired muscle group, the device has a surface area of from about 5 to about 40 sq. in. Preferably, the surface area of the device is from about 8 to about 35 sq. in., more preferably, from about 15 to about 25 sq. in., and even more preferably, from about 18 to about 22 sq. in. In one particular embodiment, the surface area of the thermal treatment device is about 19 sq. in.

Desirably, the specific dimensions optimally cover the left or right trapezius muscle in the neck. Alternatively, the same specific dimensions may be rotated ninety (90) degrees to cover the left or right paraspinal muscles in the lower back and the common tender point of the respective sacroiliac joint. In one particular embodiment, the thermal treatment device is substantially shaped as a trapezoid or a truncated right triangle.

When the thermal treatment device is designed to be substantially trapezoidal shaped, the device has a height from about 5 inches to about 8 inches (preferably, from about 6 inches to about 7 inches, more preferably, about 6.5 inches), a base from about 2.75 inches to about 5.75 inches (preferably, from about 3.25 inches to about 5.25 inches, more preferably, about 4.25 inches), a hypotenuse from about 5.5 inches to about 8.5 inches (preferably, from about 6 inches to about 8 inches, more preferably, about 7.0 inches), and a width at the tip (i.e., side opposite the base) from about 0.5 inches to about 2.5 inches (preferably, about 1 inch to about 2 inches, more preferably, about 1.5 inches). In one embodiment, the device may have a height to base ratio of from about 1.25 to about 2.3, e.g., about 1.5. In another embodiment, the device may have a height to tip width ratio of from about 2.5 to about 7, e.g., about 4.3.

The thermal treatment device may be constructed from a disposable, breathable, non-woven fabric. Optionally, the thermal treatment device may include an adhesive backing and/or an air permeable material. In one embodiment, the device is configured to have an air permeable front material connected to an adhesive backing. In another embodiment, an air permeable front material and an adhesive backing enclose and contain an air activated exothermic reaction mixture.

In one embodiment, the thermal treatment device of the present invention comprises a thermally active component and a thermally conductive component. The thermally active component delivers heat or cold for therapeutic purposes. The thermally conductive component improves the efficiency of delivery of the heat or cold, enhancing the experience of the user.
In another embodiment, the thermal treatment device includes a thermal reservoir comprising a thermal composition. The thermal composition includes a thermal component, a material or combination of materials that activates upon the addition of heat or cold, thereby retaining the heat or cold; and combinations thereof. In one embodiment, the thermal reservoir comprises an enclosure for the thermal composition. See, e.g., Figure 1D and Figure 2D.

In one embodiment, the thermal reservoir includes an enclosure, and the thermally conductive component is attached to a surface of the enclosure.

The thermal reservoir comprises a thermal composition that can be any suitable material for either generating or holding heat or for generating or maintaining a low (cold) temperature. In one embodiment, the thermal composition emits heat from about 1 to about 10°C above the skin surface temperature of a human, when worn next to the skin of a human. Alternatively or in addition, the thermal composition has a temperature of from about 1 to about 100°C lower than that of the surface of human skin. In one embodiment, the thermal composition is a fill material that maintains a temperature from about 1 to about 100°C lower than the skin surface temperature of a human.

In one particular embodiment, the thermal reservoir comprises thermal fill materials that are a mixture of substances that react exothermically. For example, several commercial hand warmers and therapeutic heat products contain an iron powder based mixture that liberates heat as the iron is oxidized upon exposure to air. These types of systems are described in detail, for example, in U.S. Patent No. 5,918,590. It is known in the art to formulate these mixtures to maintain a temperature of at least about 40°C for at least 4 hours, and up to 24 hours. Depending upon the application or desired product design, the temperature may be maintained for at least about 8 hours, at least about 10 hours, at least about 12 hours, at least about 16 hours, or whatever time period desired.

In another embodiment, the thermal reservoir comprises a thermal composition, which includes a microwavable heat retaining material. Suitable heat retaining materials include, for example, rice, corn, barley, cherry stones, starch-based synthetic pellets, and the like. Such materials typically retain a suitable level of heat for about 20 to about 60 minutes.

In another embodiment, the thermal reservoir comprises a thermal composition that includes electrically heated or electrically cooled articles, such as a resistive heater, or a thermoelectric based cooling and heating element, such as a Peltier element.

In certain embodiments, the temperature change/contrast between an individual's skin and the thermal treatment device when measured by a thermocouple is 38°C, 40°C, 41°C, 45°C, or 50°C.

In one embodiment, the thermal composition includes a thermal fill material which may comprise iron powder. The thermal fill material may maintain a temperature of at least about 40°C for a period of from about 4 to about 16 hours when worn next to the skin of a human. Alternatively, the thermal fill material may maintain a temperature of at least about 40°C for a period of at least about 10 hours when worn next to the skin of a human.

In another embodiment, the thermal reservoir comprises a thermal fill material that is a freezable liquid or gel at room temperature. Upon storage in a freezer, the material solidifies and maintains a temperature of less than about 5°C for about 20 to about 90 minutes. In one such embodiment, the temperature measured by a thermocouple inserted between the individual's skin and the thermally conductive member of the thermal insert of this invention is 5°C, 10°C, 20°C, 25°C, or 30°C.

In one embodiment the thermal reservoir is a material or combination of materials (also termed the thermal composition) which are solid at temperatures from about -20°C to 20°C, or at about 0°C. In another embodiment, the thermal reservoir is substantially free of materials that are combustible, flammable, or volatile. As used herein, "substantially free" is defined as less than 1 percent by weight of the thermal reservoir. Combustible materials include, but are not limited to, fuels such as alcohols such as ethanol, methanol and butanol; or fuels such as lighter fluids, kerosene, lantern oils, and mixtures thereof.

In one embodiment, the thermal reservoir may include an enclosure. The enclosure can be any material that surrounds the thermal reservoir or the thermal composition within the thermal reservoir. In one embodiment, the enclosure is a pouch constructed of breathable non-woven fabric. In another embodiment, the enclosure is a water-tight polymer film pouch for holding a freezable liquid. In yet another embodiment, the enclosure is constructed from a woven textile fabric. In still yet another embodiment, the enclosure is a pouch having one surface formed from a relatively non-conductive fabric, and a second surface comprising the thermally conductive component.

The thermally conductive component has a thermal conductivity of at least about 10 W/mK. Preferably, a thermal conductivity of at least about 100 W/mK. In one embodiment, the thermal conductivity of the thermally conductive component ranges from about 150 W/mK to about 250 W/mK. For comparison purposes, the thermal conductivity for some representative materials are shown below:

| | |
|---|---|
| Polypropylene: | 0.12 W/mK |
| Stainless steel: | 21 W/mK |
| Aluminum: | 221 W/mK |

In one embodiment, the thermally conductive component is comprised primarily of a metal. Alternatively or in addition, the thermally conductive component may comprise at least one element selected from the group consisting of conductive textiles, composites, plastics, polymers, rubber, ceramics and mixtures thereof.

Suitable materials for forming the thermally conductive component include, for example, metals such as aluminum, copper, silver, steel, and metal alloys of aluminum, copper, silver, steel, and combinations thereof; non-metallic thermally conductive materials such as carbon-based materials, including graphite, glassy carbon, thermally conductive plastics, polymers, rubber, or such as conductive textiles, composites, ceramics, and mixtures thereof. Optionally, these thermally conductive components can contain wires or fibers comprising the metals described above in order to make them more thermally conductive. Preferably, the thermally conductive component is non-reactive with the thermal fill composition, or with air and moisture.

In one embodiment, the thermal composition comprises a microwavable heat retaining material.

In embodiments wherein the thermal reservoir comprises a thermal composition that is activated by microwave, the thermally conductive component must be designed accordingly. For example, the thermally conductive component may include a non-metallic substance, such as ceramic. Alternatively, the thermally conductive component may include a plastic portion that has a shielded metallic surface, which is not exposed to the microwave. In yet another variation, the thermally conductive component may be packaged separately from the thermally active component, along with means (e.g., such as an adhesive) for attaching the thermally conductive component to the thermal reservoir after microwave heating.

The thermally conductive component may have a portion of its surface raised above the plane of the thermally active component. In certain such embodiments, the raised portions (also termed protrusions) may have a rounded shape. As used herein, rounded shape is defined as elliptical, semi-elliptical, semi-circular, or circular. In certain such embodiments the raised portions of the conductive surface are raised by from about 2 millimeters to about 3 centimeters from the surface of the active component. The raised portions of the surface can advantageously provide a massaging sensation when held against the skin. For example, when the thermal treatment device of the present invention is worn in a back belt, with the raised portions of the thermally conductive component in close contact with the skin, the raised portions can give the sensation of fingers, massaging the skin as the user moves. In one particular embodiment, all or a portion of the thermally conductive component can be configured to rotate around a supporting element, or within a socket. In this embodiment, the thermally conductive massaging element can be shaped as a cylinder, sphere, octahedron, dodecahedron, or any suitable rotatable shape.

It should be understood that the thermally conductive component can be of a various shapes, including round, semi-spherical, elongated, ellipsoidal, cylindrical, star shaped, mushroom shaped, or similar shapes. According to an embodiment of the present invention, the shapes of the thermally conductive component at the interfaces to the individual's body can be flat or non-flat, including but not limited to semi-spherical, pyramidal, conical, concave, convex, bumped, or contain an array of smaller shapes, e.g., semi-spherical protrusions.

In one embodiment, the thermally conductive component forms a continuous layer. In an alternative embodiment, the thermally conductive component is discontinuous.

In certain embodiments, the thermally conductive component can form a single, continuous layer on the surface of the thermally active component. For example, the thermally conductive component can be a single piece of foil having deep drawn protrusions in its surface. In certain other embodiments, the thermally conductive component can be discontinuously arranged upon a surface of the thermally active component. For example, the thermally conductive component can be a single piece of foil having cut-outs to enhance aesthetics or breathability of the thermal treatment device, or the thermally conductive component can comprise a plurality of individual metallic parts, which individually adhere to the surface of the enclosure for the thermal composition. In embodiments where the thermally conductive component is a piece of foil, the thickness of the foil can be from about 0.006 mm to about 0.3 mm. Preferably, about 0.01mm to about 0.2 mm. The foil can be present on a single surface of the thermal reservoir, on two or more surfaces of the thermal reservoir or surrounding the entire thermal reservoir.

The thermally conductive component can itself form a portion of the enclosure for the thermal fill composition. For example, the thermal fill composition can be a powder enclosed in a pouch-type structure, one surface of which comprises a porous non-woven fabric, and another surface of which comprises a metallic thermally conductive material. Or in another example, the thermal fill composition can be a freezable liquid or gel enclosed in a pouch-type structure, one surface of which comprises a polymeric water-tight film, and another surface of which comprises a metallic film.

Moreover, the thermally conductive component can be all or partially contained within the enclosure for the thermal reservoir. For example, the thermally conductive component can be in the form of pellets having a diameter from about 1 to about 20 millimeters, which are dispersed throughout the thermal fill composition. Preferably, the pellets have a diameter from about 2 to about 10 millimeters.

In another such embodiment, the thermal treatment device can be configured so that a portion of the thermally conductive component is in contact with the thermal composition and the interior of the enclosure, while another portion of the thermally conductive component protrudes through openings in the enclosure to form an exterior surface.

The thermally conductive component can be rigid, or soft and compressible. When a massaging sensation is desired, the thermally conductive component includes massaging elements that are preferably rigid enough to maintain their shape when pressed against the skin. The raised portions of the thermally conductive component can be solid, hollow, or filled with conductive or non-conductive material. In one embodiment, the raised portions are provided with apertures to enable the release of one or more agents retained therein. The agents can be released either as a result of heat generated by the thermal reservoir or by the removal of one or more covering layers. In one embodiment, the interior surfaces of the raised portions of the thermally conductive component are in direct contact with the thermal fill material. In one embodiment, the thermally conductive component is filled with metal pellets.

Another aspect of the present invention relates to methods for treating or managing pain, particularly muscle or joint pain, in humans. As noted previously, heat and massage have long been recognized as effective modalities for managing pain. The thermal treatment device of the present invention may be configured such that the thermally conductive component has at least a portion of its surface raised above the plane of the thermally active component, thus providing a means for delivering heat and a massaging sensation to the user. Compared to other methods of providing heat and massage, the method of the present invention is advantageously portable, wearable, and long lasting with minimal effort required on the part of the user. An additional benefit of the massaging action of the thermal treatment device of the present invention is the further increase in blood flow to the affected area, facilitating the oxygenation, and removal of waste from the affected tissue. For example, the device may be worn for a time period of from about 1 to about 16 hours. Or if desired, from about 4 hours to about 8 hours, or from about 8 hours to about 12 hours, or from about 8 hours to about 16 hours. Thus, providing heat to the affected muscles or joints while simultaneously engaging in work or leisure activities.

In the therapeutic use of the thermal treatment device of the present invention, the thermally conductive component(s) are in contact with the body of the user, either directly contacting the skin or contacting the body through clothing or garments worn by the user. Simultaneously the thermally conductive component(s) are in contact with the thermal treatment device. The thermally conductive component(s) serve to effectively transfer or redistribute heat or cold from the thermal treatment device to the individual's body. In addition, thermally conductive component(s) create a non-uniform thermal sensations on the body or on the skin in the case of direct application to skin, whereby body or skin areas in immediate contact with the thermally conductive component experience much stronger sensations of heat or cold relative to the adjacent areas.

In one embodiment, the thermally conductive component is substantially free of activated carbon, e.g., less than 0.1 % by weight of the fill of the thermally conductive component.

In another embodiment, the interior cavities created by raised portions of the thermally conductive component are filled with substances that are capable of retaining heat for extended periods of time, such as thermal beads, encapsulated water, wax, phase change materials, ceramics, sand, grains, rice, wheat, corn, etc. Even after the chemical formulation inside the thermally active component stops delivering or generating heat, the substances that are capable of retaining heat for extended periods of time inside the thermally conductive component can continue releasing or absorbing heat for extended periods of time. Additionally, in case of accidental overheating of the chemical formulation inside the thermally active component, the substances are capable of absorbing the excess heat thus providing protection form overheating.

Advantageously and beneficially, the space around the raised portions of the thermally conductive component is available for removal and evaporation of sweat. Additionally, the thermal contrast (temperature difference between the skin and device) delivered to the body can be much higher when a thermally conductive component transferring heat and transferring cold is immediately adjacent to the body. This contrast can be achieved without significant loss of thermal energy due to heat transfer. In one embodiment, the thermal reservoir is a thermal pack.

The number of the thermally conductive component(s) per single thermal pack can vary from one to several. For example, from 6 to 30 or more thermally conductive components may be installed on one thermal pack. For example, the thermal treatment devices shown in Figure 3B and Figure 4A, each have nine conductive components. The thermally conductive components typically have a width from about 5 millimeters to about 50 millimeters or preferably, from about 7 millimeters to about 20 millimeters. The thermally conductive components may have a height from about 5 millimeters to about 50 millimeters, or preferably, from about 7 millimeters to about 20 millimeters. As best illustrated in Figure 3C and Figure 4C, the height is measured from the surface of the device to the apex of the thermal conductive component.

In embodiments wherein the shape of the thermally conductive components are semi-spherical, the diameter, which is equal to the width of the component, is from about 5 millimeters to about 50 millimeters or preferably, from about 10 millimeters to about 30 millimeters. In this embodiment the radius of the semi-spherical component, which is equal to the height, is from about 2.5 millimeters to about 25 millimeters or preferably, from about 5 millimeters to about 20 millimeters. Circular thermal conductive components are shown in Figures 3B, 3C, 4A and 4C.

In certain embodiments, the thermally conductive components can be defined by the volume of the internal space of the component. For example, the internal volume of a thermally conductive component can be from about 0.01 milliliters to about 50.00 millimeters or preferably, from about 0.03 milliliters to about 33.00 milliliters, or more preferably, from about 0.10 milliliters to about 2.00 milliliters.

The thermal treatment device may have more than one thermally conductive component. The thermally conductive components may have the same height or may be such that some thermally conductive components are higher and some are lower. For example, a first portion of the thermally conductive components may be about 5 millimeters to about 10 millimeters high, while a second portion may be about 10 millimeters to about 15 millimeters high, while an optional third portion may be about 15 millimeters to about 20 millimeters high.

In one embodiment, at least one dimension of the thermal insert is from about 1 inch to about 30 inches. In one particular embodiment, thermal reservoir 12 has a triangular shape with a width from about 2 to about 6 inches, and overall length from about 2 to about 12 inches.

In certain embodiments, the thermal treatment device can be substantially flat with the thickness of the device ranging from about 2 millimeters to about 30 millimeters, and the other dimensions of the device ranging from about 24 millimeters to about 720 millimeters.

In one embodiment, the thermal device of the present invention is worn in close proximity to the skin by placing the device in a pouch as part of an article of clothing, a belt, or a specially designed sachet.

In one embodiment, the thermal device of the present invention is worn in close proximity to the skin by using an adhesive, which may be selected from the group consisting of a hydrogel, silicon adhesives (e.g. a silicone gel), a hot melt adhesive and mixtures thereof.

The thermal treatment device may be worn in close proximity to an individual's skin or may be directly applied to the skin. When direct application is employed, an adhesive may be used. In one embodiment, the thermal device of the present invention has a first side that includes an air permeable material and a second side that includes an adhesive. Any suitable adhesive that is safe and effectively enables the thermal treatment device to adhere to the skin may be used. Suitable adhesives include, for example, hydrogels, silicone adhesives (e.g. silicone gel), hot melt adhesives, and the like or mixtures thereof. Ideally, the adhesive should permit the thermal treatment device to be applied and conform to the skin contact surface area. In some cases, the adhesive may facilitate the even distribution of heat or cold across the skin area covered by the thermal treatment device.

In one embodiment, the thermal device of the present invention is re-useable. In an alternative embodiment, the thermal device of the present invention is disposable.

.The thermal treatment device may be adapted to be inexpensive, light-weight entirely disposable and easily portable, thus allowing travel and mobility.

While the invention has been described above with reference to specific embodiments thereof, it is apparent that many changes, modifications, and variations can be made without departing from the inventive concept disclosed herein. Accordingly, it is intended to embrace all such changes, modifications, and variations that fall within the spirit and broad scope of the appended claims. All patent applications, patents, and other publications cited herein are incorporated by reference in their entirety.

## Claims

1. A thermal treatment device to be worn in close proximity to the skin of a human comprising a thermal composition, wherein the device has a shape that substantially covers a surface of the trapezius muscle or the paraspinal muscles in the lower back and the tender point of the respective sacroiliac joint.

2. The thermal treatment device of claim 1, wherein the device has a surface area of from about 8 to about 35 sq. in.

3. The thermal treatment device of claim 1 or claim 2, wherein the device is substantially trapezoidal shaped or substantially shaped as a truncated right triangle.

4. The thermal treatment device of claim 3, wherein the substantially trapezoidal shape has a height from about 5 inches to about 8 inches, a base from about 2.75 inches to about 5.75 inches, a hypotenuse from about 5.5 inches to about 8.5 inches, and a width at the tip from about 0.5 inches to about 2.5 inches.

5. The thermal treatment device of claim 3 or claim 4, wherein the substantially trapezoidal shape has a height to base ratio of from about 1.25 to about 2.3 and a height to tip width ratio of from about 2.5 to about 7.

6. The thermal treatment device of any preceding claim, further comprising a thermally conductive component.

7. The thermal treatment device of claim 6, wherein the thermally conductive component has a thermal conductivity of at least about 100 W/mK, preferably from about 150 W/mK to about 250 W/mK.

8. The thermal treatment device of claim 6 or claim 7, wherein the thermal reservoir has an exterior surface.

9. The thermal treatment device of any of claims 6 to 8, wherein the thermally conductive component has a portion of its surface that is raised above an exterior surface of the thermal reservoir.

10. The thermal treatment device of any of claims 6 to 9, wherein the conductive component has raised protrusions having a rounded shape.

11. The thermal treatment device of claim 10, wherein the raised portions have a maximum height of from about 5 millimeters to about 50 millimeters as measured from the surface of the thermal reservoir or enclosure of the thermal reservoir.

12. The thermal treatment device of claim 10 or claim 11, wherein the raised portions have rounded edges and rotate around a supporting element or within a socket.

13. The thermal treatment device of any one of the preceding claims, wherein the device is substantially trapezoidal axisymmetric shaped.

14. The thermal treatment device of any one of the preceding claims, wherein the substantially trapezoidal asymmetric shape has a height from about 4.5 inches to about 6 inches and a base from about 3.375 inches to about 4.55 inches.

15. A method for treating muscle aches and pains in a human, comprising the step of: wearing the thermal treatment device of any one of the preceding claims for a time period of about 1 hour to about 16 hours.
